# EUROPEAN PATENT SPECIFICATION

(11) **EP 3 217 882 B1**
(45) Date of publication and mention of the grant of the patent: **15.01.2020**
(21) Application number: 15804589.8
(22) Date of filing: 05.11.2015
(51) Int. Cl.: A61B 6/04, A61B 6/00

(54) **RADIOLOGICAL APPARATUS WITH A TABLE MOVEMENT SYSTEM WITH VARIABLE ANGLE RODS**
RADIOLOGISCHE VORRICHTUNG MIT TISCHBEWEGUNGSSYSTEM MIT VARIABLEN WINKELSTANGEN
APPAREIL RADIOLOGIQUE AVEC UN SYSTÈME DE DÉPLACEMENT DE TABLE AVEC TIGES À ANGLE VARIABLE

(30) Priority: 11.11.2014 IT CO20140035
(43) Date of publication of application: 20.09.2017
(73) Proprietor: General Medical Merate S.p.A., 24068 Seriate (BG) (IT)
(72) Inventor: SCARPELLINI, Luciano, 24068 Seriate (BG) (IT)
(74) Representative: De Ros, Alberto
(86) International application number: PCT/IB2015/058547
(87) International publication number: WO 2016/075594

(56) References cited:
- US-A- 4 484 343
- US-A1- 2012 023 671
- US-A1- 2012 198 624

## Description

The present invention relates to a radiological apparatus with a table movement system with variable angle rods.

In the field of radiological apparatuses, the technicians are always looking for new, simple and effective solutions to move the table, preferably with great flexibility of movement and positioning.

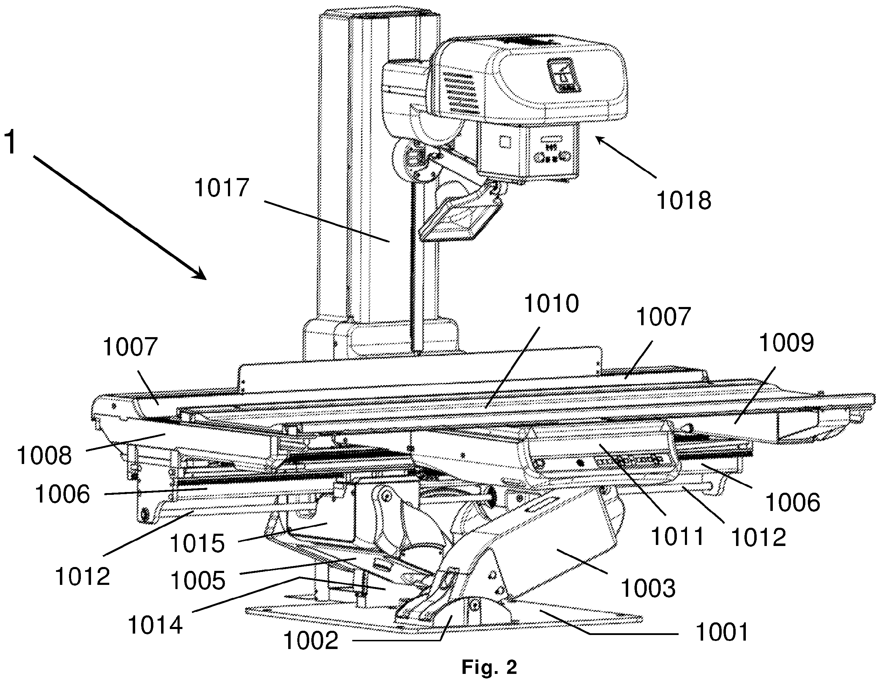

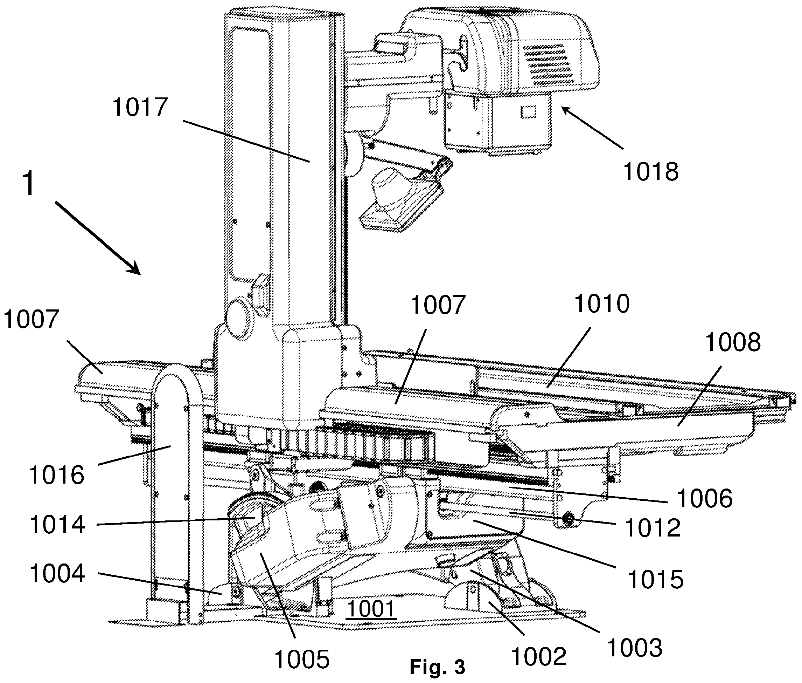

A radiological apparatus is known from patent document U.S. 2012/0023671 A1, in which radiological apparatus the movement of the table is implemented by means of two rods arranged obliquely and mutually hinged at a central point thereof; a first rod (5 in Fig. 2 and Fig. 3) of the two rods is hinged, at a lower end thereof, to a first fixed point of the base of the apparatus; a second rod (6 in Fig. 2 and Fig. 3) of the two rods is hinged, at a lower end thereof, to a point of the carriage sliding on the base of the apparatus. According to this embodiment, the table is and remains always horizontal.

The Applicant has the general object of finding a simple, effective and flexible solution.

Such a general object is achieved by the radiological apparatus having the technical features set out in the appended claims, which are to be considered as an integral part of the present description.

The general idea underlying the present invention is to implement the movement of the table by means of two rods that are hinged to a base so as to have a variable position; the two rods are arranged in an oblique manner, mutually opposite and crossed, but are not hinged to each other; they are constrained to the table by means of two hinges and two carriages, respectively. It follows that the table has a variable position that adapts to the operating conditions (i.e. the position and mobility of the patient and the examination to be made) and to the installation conditions of the radiological apparatus (i.e. the installation position of the apparatus with respect to the diagnostic room and with respect to the people and means that are and move therein).

The present invention (from the structure, function and use point of view) as well as its advantages will become more apparent from the following detailed description, to be considered together with the accompanying drawings, in which:
Fig. 1 shows a schematic front view of a possible system for moving the table of a radiological apparatus according to the present invention,
Fig. 2 shows a three-dimensional front view of a specific exemplary embodiment of a radiological apparatus according to the present invention,
Fig. 3 shows a three-dimensional back view of the apparatus in Fig. 2,
Fig. 4 shows a two-dimensional front view of the apparatus in Fig. 2,
Fig. 5 shows a schematic view of a possible spring actuator for a radiological apparatus according to the present invention,
Fig. 6 shows a sectional view of a spring actuator for the apparatus in Fig. 2 in three different operating positions,
Fig. 7 shows the apparatus in Fig. 2 in a first operating position,
Fig. 8 shows the apparatus in Fig. 2 in a second operating position,
Fig. 9 shows the apparatus in Fig. 2 in a third operating position, and
Fig. 10 shows the apparatus in Fig. 2 in a fourth operating position.

Both such a description and such drawings are to be considered only for illustrative and therefore non-limiting purposes.

As is easily understood, there are various ways to implement the present invention in the practice, which is defined in the appended claims.

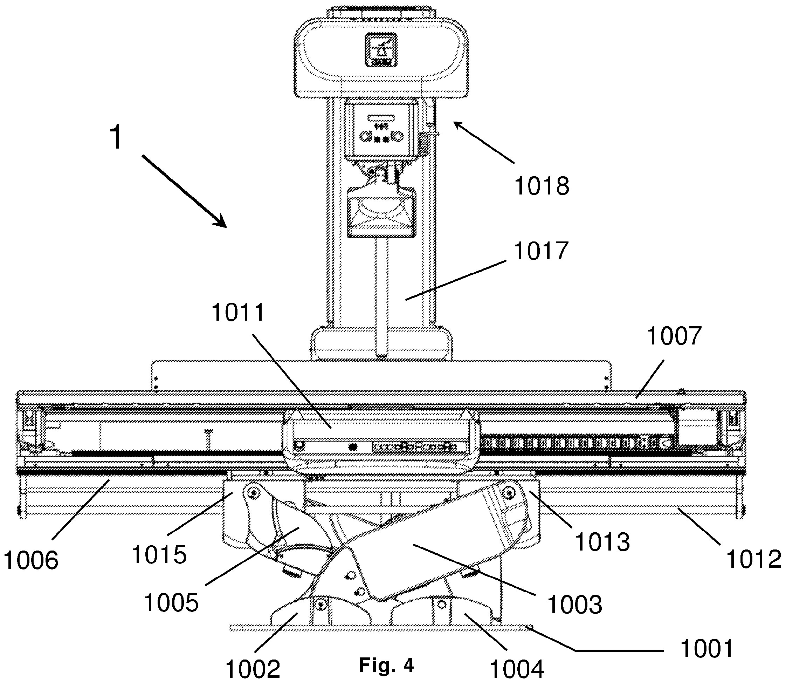

Figures from Fig. 2, Fig. 3, Fig. 4 and Fig. 6 refer to the same specific exemplary embodiment of a radiological apparatus (indicated with reference numeral 1 as a whole).

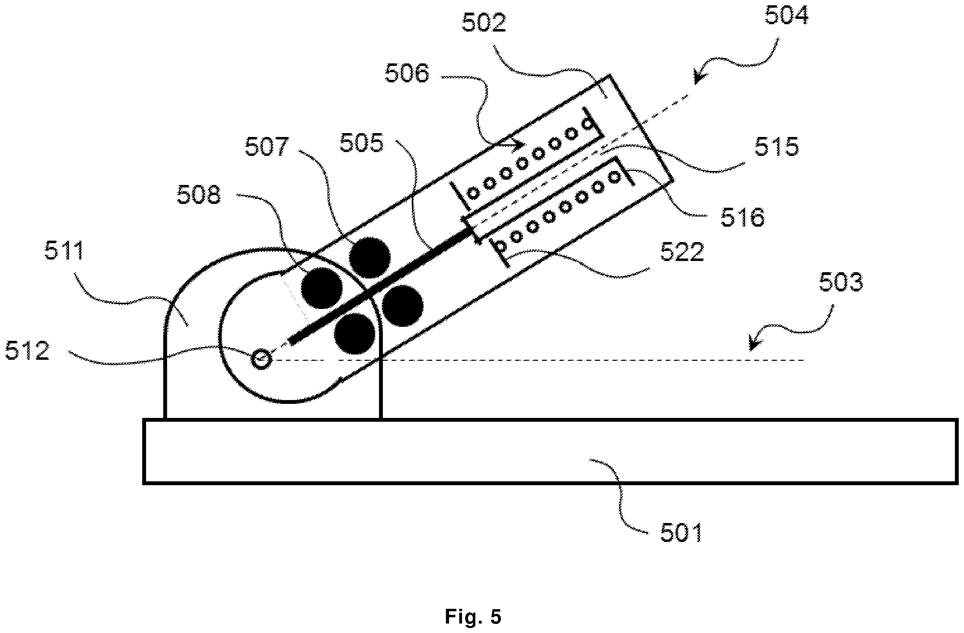

Fig. 5 schematically shows a component of the radiological apparatus 1 in figures 2-4 and 6. The figure is intended to effectively describe the operation of such a component.

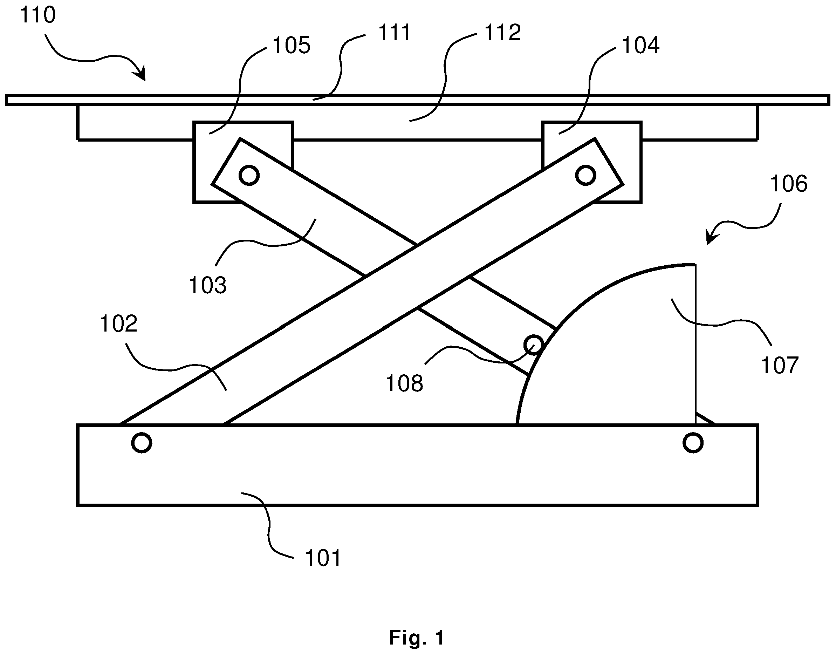

The solution shown schematically in Fig. 1 serves only to explain the present invention and is substantially implemented in the radiological apparatus 1.

Of course, there is no total and perfect correspondence between the diagrams in figures 1 and 5 and the solution in figures 2, 3, 4 and 6.

Fig. 1 is a schematic figure that allows understanding the general idea underlying the present invention (and its more specific ideas) which relates to the movement of the table of a radiological apparatus.

Fig. 1 shows a base 101 (which is rigid and is adapted to be resting on a floor), a table 110 with a patient "plane" 111 and with a rail 112 (which at the same time forms both a first rail and a second rail, as will be understood hereinafter), and a system for moving table 110; the movement system essentially comprises: a first rod 102 (which is rigid), a second rod 103 (which is rigid), a first carriage 104 (which is motorized), a second carriage 105 (which is motorized), and constraining means 106 or "constraint(s)".

The first rod 102 is hinged at a first end to a first point of base 101 and at a second end to the first carriage 104, the second rod 103 is hinged at a first end to a second point of base 101 and at a second end to the second carriage 105; the distance between the first and the second points of the base is fixed and predetermined.

The two rods 102 and 103 are directly mutually unconstrained, i.e. there are no direct mechanical constraints between them, and thus, in particular, they are not hinged to each other; the two rods 102 and 103 are arranged obliquely, mutually opposite and crossed; moreover, they are arranged completely underneath the table, in particular underneath the patient plain.

The first carriage 104 is adapted to slide along a first rail of table 110; the second carriage 105 is adapted to slide along a second rail of table 110; however, according to Fig. 1 and according to an advantageous embodiment of the present invention, they coincide with the single rail 112. It should be noted that the term "rail" is used herein, but the term "guide" may also be used with almost similar meaning.

The motorization of the two carriages 104 and 105 consists in that each of these is equipped with a motor (typically electric) which allows them to translate along their respective rails (in particular along the same rail).

It is understandable therefore that the position of table 110 and, in particular, of the patient plane 111 depends on the positions of the two carriages 104 and 105. It is understood that the table 110 includes the patient plane 111, and the movement of the table 110 and patient plane 111 assembly is carried out by means of carriages 104 and 105. Considering Fig. 1 (which is a schematic front view), by means of the positioning of the two carriages it is possible to determine each of the inclination angles of the two rods and thereby the positioning of table 110 and of its patient plane 111; the position or setting of the table (in particular of the table in Fig. 1) can be defined by three parameters: vertical position (i.e. Y coordinate) of its center of gravity, horizontal position of its center of gravity (i.e. X coordinate), angular position of the patient plane 111 with respect to axis X.

The combined movements of the two carriages (in particular along the same rail) allow obtaining the "translation", "elevation" and "tilting" of the table. In particular: if the carriages move at the same speed, direction and orientation, the "translation" is obtained (with reference to Fig. 1, the table translates rightwards or leftwards), if the carriages move at the same speed and direction, but in opposite orientation, the "elevation" is obtained (with reference to Fig. 1, the table translates upwards or downwards), if the carriages move in the same direction, but at different speed/orientation, the "tilting" is obtained (with reference to Fig. 1, the table rotates).

According to the preferred embodiment, the operator controls the positioning of table 110, which includes the patient plane 111, consequently he also controls the positioning of the patient plane 111. The motor associated with carriage 105 acts as "master" and the motor associated with carriage 104 acts as "slave".

It follows that the table has a variable position that allows the apparatus to adapt to its operating conditions (i.e. the position and mobility of the patient and the examination to be made) and to its installation conditions (i.e. the installation position of the apparatus with respect to the diagnostic room and with respect to the people and means that are and move therein). In order to stabilize the position of table 110, constraining means mentioned above are also provided which serve for constraining the rotation of at least one of the two rods; in the example in Fig. 1, the constraining means serve to directly constrain only one of the two rods, in particular the second rod 103.

Fig. 1 schematically shows also the constraining means 106 according to a particularly advantageous embodiment thereof. Means 106 comprise a circular arc 107 of toothed wheel fixed to base 101 and a rotating toothed pin 108 meshed on the toothed wheel 107; pin 108 is mounted to the shaft of a motor (typically electric) which is fixed to only one of the two rods (in particular, rod 103); this motor is interlocked to the motor associated with one of the two carriages (in particular, carriage 105 of the same rod 103).

The motors (typically electric) of the apparatus are typically controlled by a computerized control unit (which is not shown in the figures and which advantageously will also have other functions) in such a way that it is easy for the operator to obtain the desired positioning of table 110 (and, as mentioned, also of the patient plane 111).

Of course, the apparatus according to the present invention comprises an X-ray emitter and an X-ray receptor (which are shown only in Fig. 2 and Fig. 3 and Fig. 4). The X-ray receptor can be mounted to the table and be adapted to translate in a longitudinal direction with respect to the patient plane 111 (as is the case of the example in Fig. 2 and Fig. 3 and Fig. 4). In particular, the apparatus may comprise a stator in the form of column (perpendicular to the patient table) mounted to the table and adapted to translate in a direction parallel to the median axis of the patient; in this case, the X-ray emitter can be mounted (indirectly) to the stator.

Fig. 5 is a schematic figure that allows understanding the general idea underlying a spring actuator (and its more specific ideas) usable in a radiological apparatus according to the present invention. It is noted that in the diagram in Fig. 5, the internal spring to the actuator (indicated with reference numeral 506) exerts force as a result of the compression of the same spring.

Such a spring actuator serves to apply a torque on only one of the rods of the movement system of table 110 of a radiological apparatus. In particular, with reference to the figures, the actuator acts on the rod (102 in Fig. 1) that is not associated with constraining means (106 in Fig. 1).

The rod associated with the actuator is typically adapted to rotate along an angular stroke between a first end angular position (for example corresponding to a position inclined by 10° with respect to the horizontal 503, or 5° or 15°) and a second end angular position (for example corresponding to a position inclined by 115° with respect to the horizontal 503, or 120° or 110°); in this case, the actuator is made in such a way that:
- it does not apply torque when the rod is in a predetermined intermediate angular position 504, or neutral position, between the first end angular position and the second end angular position (for example, is inclined by 62.5° or 50° or 75° with respect to the horizontal),
- it applies torque directly towards this predetermined intermediate angular position 504 when the rod is in the first end angular position,
- it applies torque directly towards this predetermined intermediate angular position 504 when the rod is in the second end angular position,
in other words, the actuator favors the return movement of the rod to the intermediate angular position or neutral position.

The actuator can be implemented in such a way that the torque it applies varies depending on the angular position of the rod, in particular on the distance of the rod from the intermediate angular position or neutral position.

Moreover, the actuator is typically adapted to accumulate energy, or "preload", both when the rod rotates from the predetermined intermediate angular position or neutral position to the first end angular position and when the rod rotates from the predetermined intermediate angular position or neutral position to the second end angular position; in other words, the energy used by the actuator to return the rod to the neutral position is accumulated during the step of moving the rod away from the intermediate angular position or neutral position.

The actuator in Fig. 5 can therefore accumulate energy, i.e. "preload", during the rotations of the rod in a clockwise and counter clockwise direction with respect to the intermediate angular position or neutral position, and then return the accumulated energy due to at least one mechanical elastic element or mechanical spring (reference 506 in Fig. 5); therefore, such an actuator will produce a significant reduction of the torques required by the electric motors for the movement of rods 102, 103, and thus of table 110.

Fig. 5, the actuator comprises:
- a first portion (essentially elements 507 and 522) which is integral with the rod (element 502),
- a second portion (essentially elements 508 and 512) which is integral with the base (element 501, which is the plate of the base and element 511, which acts as a hinge pin which allows the rotation of rod 502),
- a bending but substantially inextensible linear element, in particular a chain 505, which has a first end mechanically connected with the first portion (in Fig. 5, such a connection with element 522 is indirect through the stem 115 and mechanical spring 506 assembly) and with the second portion (in Fig. 5, such a connection with element 512 is direct),
- a stem 515, provided with a ring nut at a first end; the second end of the rod (opposite the first) is fixed to an end of chain 505,
- a spring 506 which works in compression; the spring surrounds the shank of stem 515,
- a lever body 522 integral with the rod and through which stem 515 can slide.

A first end portion of spring 506 is in contact with the ring nut; a second end portion of spring 506, opposite the first, is in contact with the lever body 522. Spring 506 is therefore compressed between the ring nut 515 and the lever body 522.

In Fig. 5, element 505 is located between elements 507, which are two guides in the shape of a pin, of the portion integral with the rod and between elements 508, which are two guides in the shape of a pin, of the portion integral with the base (and thus fixed).

Fig. 5 corresponds to the neutral position of rod 502. When a rotation of rod 502 from the neutral position (either clockwise or counter clockwise) is caused, element 505 bends because it is forced to remain between the two pins 507 and between the two pins 508 irrespective of the position of rod 502. Such a rotation causes pins 507 to move away from pins 508; moreover, the stem slides and compresses spring 506 which accumulates energy. The compressed mechanical spring 506 exerts a force on element 505 and, due to pins 507 and 508, a moment on rod 502 (with respect to base 501) which tends to bring it back to the neutral position.

From the above, it is clear that the system for moving the patient table of a radiological apparatus according to the present invention (which includes two, and preferably three motors, typically electric) is improved by the presence of at least one spring actuator.

Fig. 5 only serves for explaining the operating principle of a spring actuator according to the present invention.

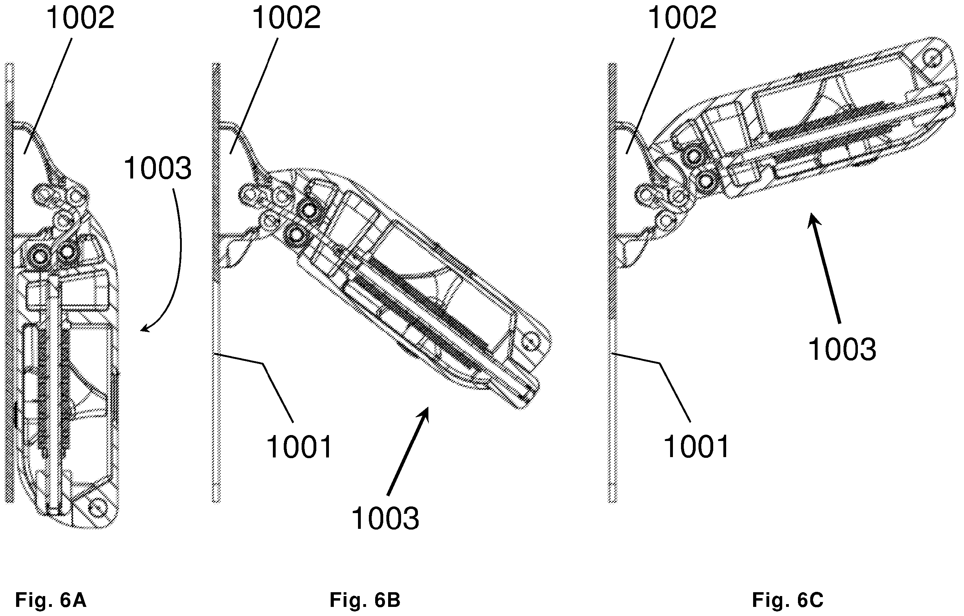

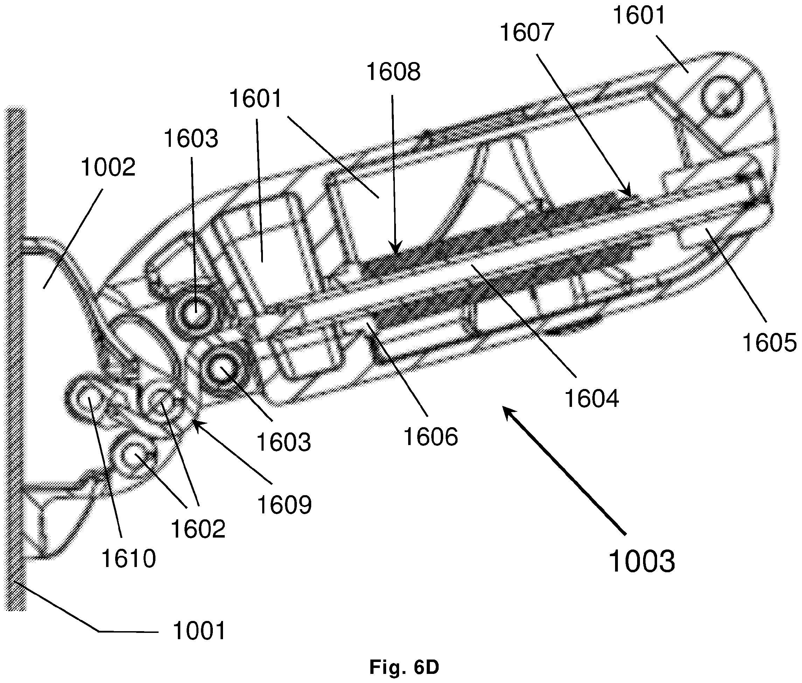

Fig. 6 serves for further explaining the operating principle of a spring actuator as described in figure 5 according to the present invention. Fig. 6 shows a sectional view of the spring actuator for the apparatus in Fig. 2 in three different operating positions; Fig. 6B corresponds to the neutral position (in fact, the chain is straight), also referred to as "predetermined intermediate angular position"; Fig. 6A corresponds to the "first end angular position"; Fig. 6B corresponds to the "second end angular position"; Fig. 6D is identical to Fig. 6C, but in enlarged scale and with reference numerals added.

As described, in the actuator in Fig. 6, one end of the bending linear element is connected with the end of a stem (in particular straight) that can slide along (in particular within) a guide (in particular straight) that is integral with the rotating rod; the stem is connected with an elastic mechanical element which consists in particular by a series of cup springs (inserted around the guide); such an elastic mechanical element opposes the translation of the stem in the direction of the fulcrum of the rotating rod, that is, it is compressed by the movement of the bending linear element and by the consequent translation of the stem.

From the above, it is clear that the system for moving the table of a radiological apparatus according to the present invention (which includes two, and preferably three motors, typically electric) is improved by the presence of at least one spring actuator.

Moreover, it is clear that the configuration of the actuator shown in figures 5 and in figures 6 is a particularly advantageous embodiment. However, it is possible to implement an actuator similar to that described, in which the elastic mechanical element works in traction and, for example, the linear element is directly connected to the elastic mechanical element.

Finally, the "parking" of the apparatus, i.e. a positioning of its components (including the table) that facilitates maneuvers with other devices (such as stretchers and moving beds) may be provided, among other things, right in the room where the apparatus is present.

According to an embodiment, the movement of the apparatus in such a "parking" position provides for movements in opposite directions of table 110 with respect to movements of the stator and the receptor assembly. When an operator sets the "parking" position of apparatus 1 (for example by the computerized control unit), table 110 and the stator and receptor assembly move simultaneously and according to opposite directions; for example, if the table 110 is positioned to the right, the stator and receptor assembly is positioned to the left, and if the table 110 is positioned to the left, the stator and receptor assembly is positioned to the right.

For rooms of modest size, it is also possible to use the apparatus with the table moved to the right and the stator and receptor assembly positioned to the left. In this configuration, only some of the movements of the apparatus are possible.

The system for moving the table such as that described in general with reference to Fig. 1 (and Fig. 5), essentially provides three aspects:
- possibility of tilting the table up to +/- 90°
- elevation of the table with respect to the base
- translation of the table with respect to the base.

Especially the first two functional aspects meet operational requirements for the positioning of the patient, while the third aspect, in addition to the functional aspect, allows for the optimization of the spaces within the diagnostic room where the apparatus is installed, in fact the translation to the right frees spaces useful, for example, for moving stretchers and/or bedridden patients, as well as use of the apparatus in rooms of modest size.

As already mentioned, Fig. 2, Fig. 3, Fig. 4 and Fig. 6 relate to the same specific exemplary (and advantageous) embodiment of a radiological apparatus 1.

Apparatus 1 rests on a base plate 1001 to which a first support 1002 for a front lever 1003 and a second support 1004 for a back lever 1005 are attached; lever 1003 is inferiorly pivoted on support 1002 and lever 1004 is inferiorly pivoted on support 1004.

In apparatus 1 there is a structure for supporting the patient which comprises a frame which essentially consists of a lower longitudinal bar 1006 and an upper longitudinal bar 1007 fixed to each other by two lateral cross-members 1008 and 1009. A patient support table 1010 is attached to the two lateral cross-members 1008 and 1009. The frame and patient support table assembly can also be referred to as "table". An X-ray receptor 1011 (known per se) is mounted to bar 1006 in such a way as to slide along the same; in particular, receptor 1011 is mounted to the upper part of bar 1006. A worm screw 1012 is fixed and is attached to the cross-members 1008 and 1009: a first end thereof to cross-member 1008 and a second end thereof to cross-member 1009.

In apparatus 1 there are a first carriage 1013 and a second carriage 1015; lever 1003 is hinged to carriage 1013 and lever 1005 is hinged to carriage 1015; carriage 1013 and carriage 1015 are mounted to bar 1006 in such a way as to slide along the same independently of each other; in particular, carriages 1013 and 1015 are mounted to the lower part of bar 1006.

The patient's weight rests on the support structure, which rests on carriages 1013 and 1015, which rest on levers 1003 and 1005, which rest on the base plate 1001, which rests on the floor of the room where apparatus 1 is placed.

The base plate 1001 is also attached to a toothed sector 1014 with a horizontal axis; lever 1005 encloses sector 1014 being an element that has a shape similar to a "U" and which can rotate about the axis of the sector.

Apparatus 1 is provided with three electric motors, independent of one another but suitably controlled; one is inside lever 1005; one is inside carriage in 1013; one is inside carriage 1015. The motor inside lever 1005 rotates a gear that meshes with the toothed sector 1014; the motor inside carriage 1013 rotates an internal nut screw which is coupled to the worm screw 1012; the motor inside carriage 1014 rotates an internal nut screw which is coupled to the worm screw 1012.

A spring actuator is located inside lever 1003 which will be explained hereinafter with reference to Fig. 6D.

In Fig. 2, Fig. 3 and Fig. 4, the following is visible, inter alia:
- a cable holder column 1016 attached to the base plate 1001,
- a stator 1017 in the shape of column (perpendicular to the patient table 1010) mounted to bar 1007 and adapted to translate according to a direction parallel to the median axis of the patient support table 1010 - such a movement is carried out due to an electric motor;
- an X-ray emitter 1018 (known per se) mounted to stator 1017 (in a per se known manner).

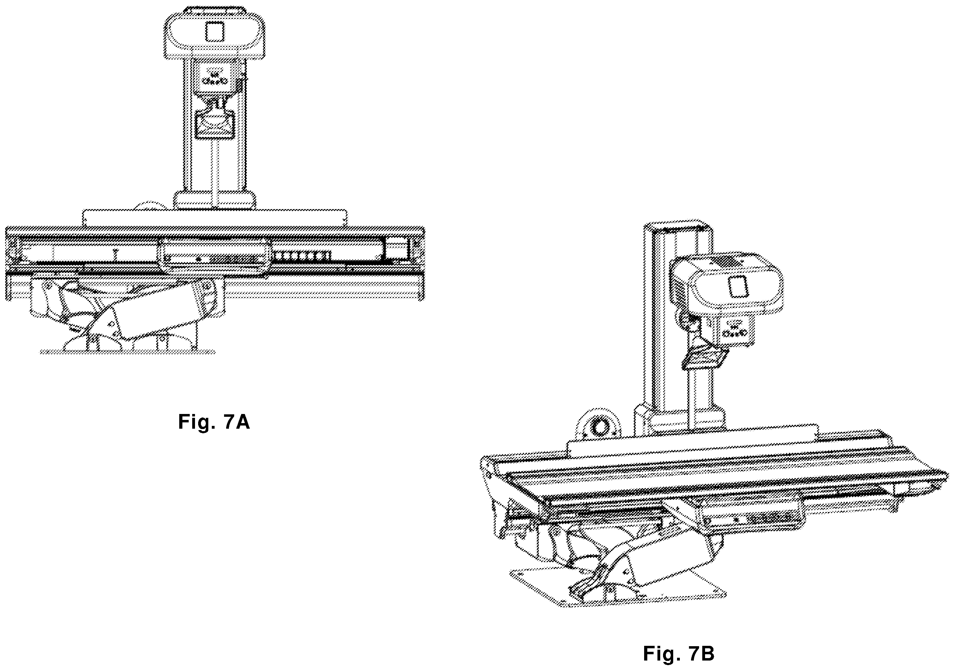

Fig. 7 shows apparatus 1 in a first operating position; Fig. 7A is a front view and Fig. 7B is a three-dimensional view; the patient support structure is horizontal and moved all to the right with respect to the base plate; the stator is vertical and at the center with respect to the patient support structure.

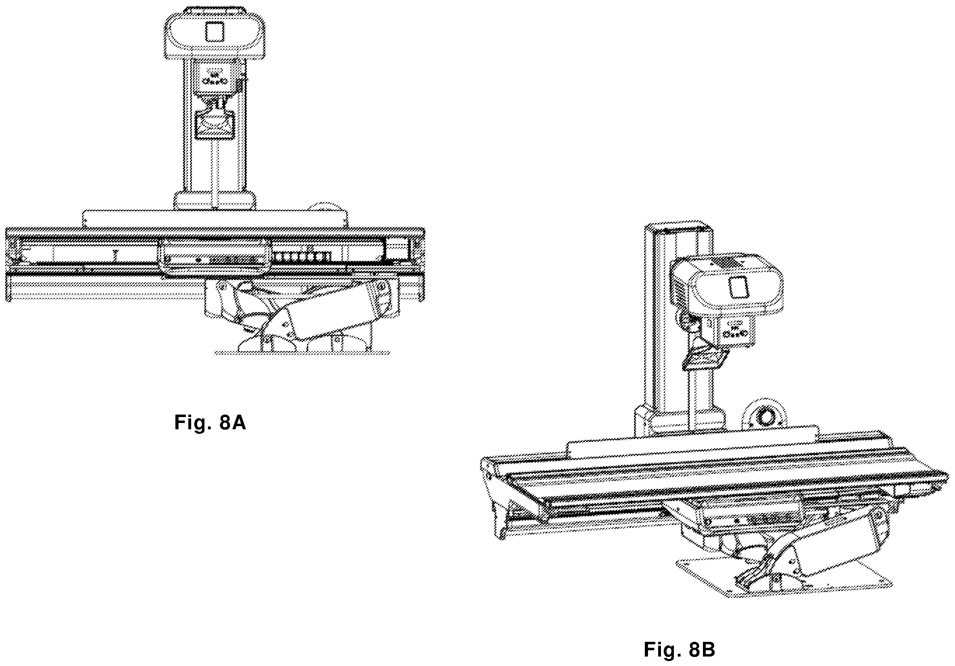

Fig. 8 shows apparatus 1 in a second operating position; Fig. 8A is a front view and Fig. 8B is a three-dimensional view; the patient support structure is horizontal and moved all to the left with respect to the base plate; the stator is vertical and at the center with respect to the patient support structure.

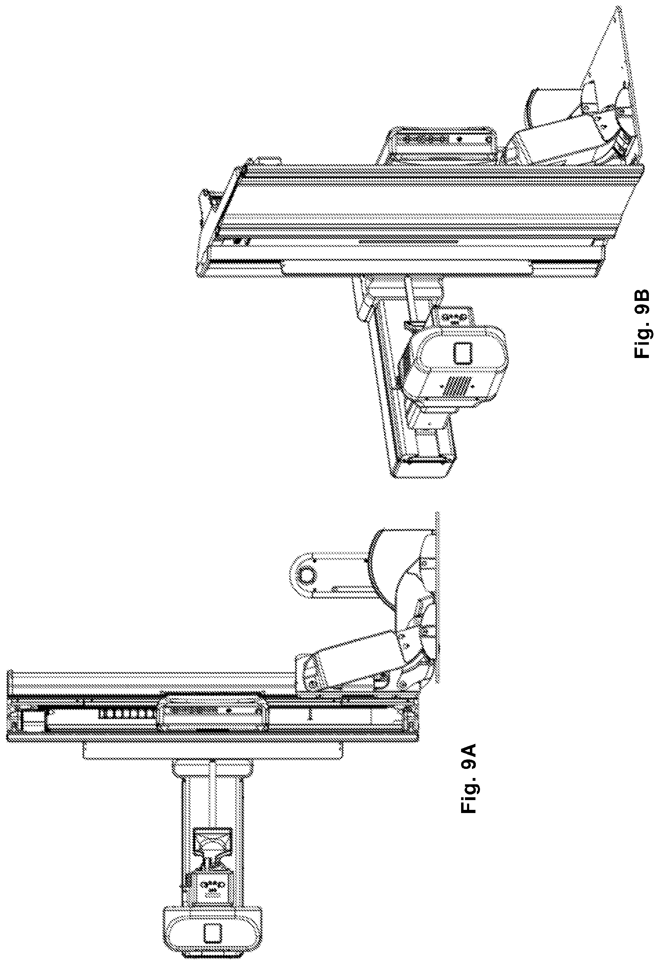

Fig. 9 shows apparatus 1 in a third operating position; Fig. 9A is a front view and Fig. 9B is a three-dimensional view; the patient support structure is vertical and to the left with respect to the base plate; the stator is horizontal and at the center with respect to the patient support structure.

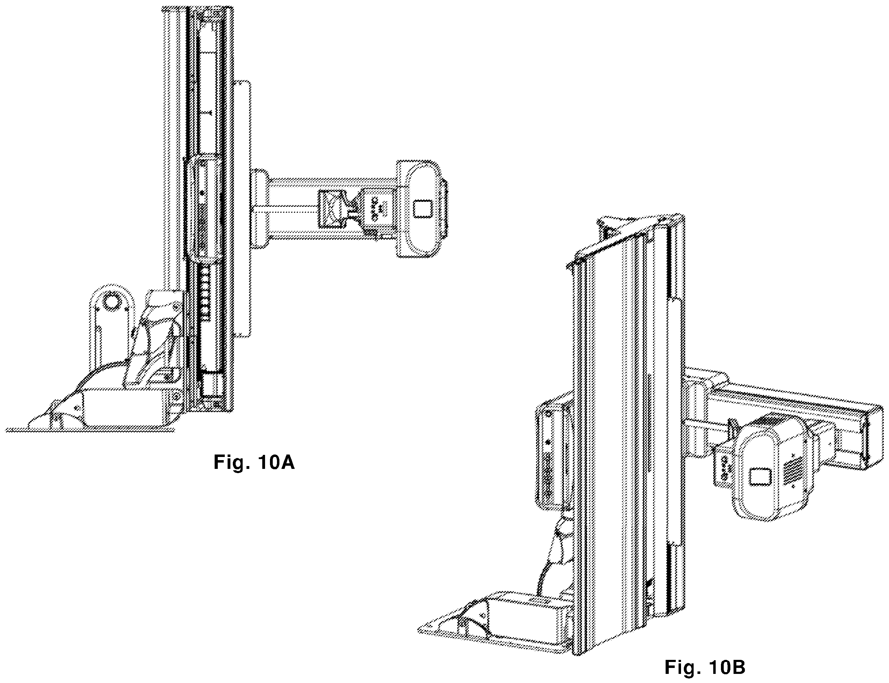

Fig. 10 shows apparatus 1 in a fourth operating position; Fig. 10A is a front view and Fig. 10B is a three-dimensional view; the patient support structure is vertical and to the right with respect to the base plate; the stator is horizontal and at the center with respect to the patient support structure.

Fig. 6D shows (in section) lever 1003 (in an operating position thereof) which is pivoted on support 1002 which is attached to the base plate 1001; lever 1003 is provided with a hollow body 1601.

Two constraining rollers 1602 are mounted in support 1002 and two constraining rollers 1603 are mounted in lever 1003.

Lever 1003 comprises a stem 1604 which can slide within body 1601; at a first end, at a first end zone, stem 1604 is provided with a guide cylinder 1605 which can slide in a first internal hole of body 1601; at a second end zone, stem 1604 can slide inside a guide bushing 1606 fixed in a second internal hole of body 1601; little beyond cylinder 1605, there is a ring nut 1607 that is screwed on stem 1604. A series of cup springs 1608 are inserted on stem 1604 and compressed between bushing 1606 and the ring nut 1607.

Lever 1003 comprises a chain 1609; a first end thereof is attached to a pin 1610 of support 1002; a second end thereof is attached to a second end of stem 1604; chain 1609 slides between the two rollers 1602 and between the two rollers 1603. Depending on the operating position of lever 1003, lever 1003 is more or less inclined, chain 1609 is more or less arched, stem 1604 is more or less projecting from body 1601, and springs 1608 are more or less compressed.

According to a specific embodiment, lever 1003 is long about 700 mm and the stroke of stem 1604 is about 60 mm.

## Claims

1. Radiological apparatus comprising a base (101) adapted to be resting on a floor, an X-ray emitter, an X-ray receptor, a table (110) with a patient plane (111) and a movement system for said table; wherein said movement system comprises:
- a first rod (102) hinged at a first end to a first fixed point of said base (101),
- a second rod (103) hinged at a first end to a second fixed point of said base, said second point being at a predetermined and fixed distance from said first point,
- a first motorized carriage (104) adapted to slide along a first rail of said table,
- a second motorized carriage (105) adapted to slide along a second rail of said table, said second rail being distinct from or coinciding with said first rail; wherein a second end of said first rod (102) is hinged to said first carriage (104);
wherein a second end of said second rod (103) is hinged to said second carriage (105); and further comprising means (106) adapted to constrain the rotation of said first rod (102) and/or of said second rod (103);
**characterized by** comprising a spring actuator (506) adapted to apply torque on said first or second rod;
wherein said first or second rod is adapted to rotate along a rotatory stroke between a first end angular position and a second end angular position,
wherein said actuator:
- does not apply torque when said first or second rod (102, 103) is in a predetermined intermediate angular position between said first end angular position and said second end angular position,
- applies torque directed toward said predetermined intermediate angular position when said first or second rod (102, 103) is in said first end angular position,
- applies torque directed toward said predetermined intermediate angular position when said first or second rod (102, 103) is in said second end angular position; and wherein said actuator (506) is adapted to accumulate energy when said first or second rod rotates from said predetermined intermediate angular position to said first end angular position and when said first or second rod (102, 103) rotates from said predetermined intermediate angular position to said second end angular position.

2. Radiological apparatus according to claim 1, wherein said first rod (102) and said second rod (103) are directly unconstrained between each other.

3. Radiological apparatus according to claim 1 or 2, wherein said first rail and said second rail are made in a single rail (112).

4. Radiological apparatus according to claim 1 or 2 or 3,
wherein said first motorized carriage (104) comprises a first electric motor;
wherein said second motorized carriage (105) comprises a second electric motor;
wherein said means (106) comprise a third electric motor interlocked to said first or second electric motor.

5. Radiological apparatus according to claim 4, wherein said means (106) comprise a circular arc of toothed wheel fixed to said base (101), and wherein said third motor is provided with a rotating shaft with a toothed pin meshed on said toothed wheel.

6. Radiological apparatus according to any one of the preceding claims, comprising further a computerized control unit adapted to control said first electric motor, said second electric motor, and said third electric motor.

7. Radiological apparatus according to any one of the preceding claims, wherein said X-ray emitter is mounted indirectly to said table.

8. Radiological apparatus according to any one of the preceding claims, wherein said X-ray receptor is mounted directly to said table.

## Patentansprüche

1. Röntgengerät, umfassend eine Basis (101), die dazu geeignet ist, auf einem Boden zu stehen, einen Röntgenstrahler, einen Röntgenrezeptor, einen Tisch (110) mit einer Patientenebene (111) und ein Bewegungssystem für den Tisch; wobei das Bewegungssystem Folgendes umfasst:
- einen ersten Stab (102), der mit einem ersten Ende an einem ersten Festpunkt der Basis (101) angelenkt ist,
- einen zweiten Stab (103), der mit einem ersten Ende an einem zweiten Festpunkt der Basis angelenkt ist, wobei der zweite Punkt sich in einem vorgegebenen festen Abstand vom ersten Punkt befindet,
- einen ersten motorbetriebenen Schlitten (104), der dazu geeignet ist, längs einer ersten Schiene des Tisches zu gleiten,
- einen zweiten motorbetriebenen Schlitten (105), der dazu geeignet ist, längs einer zweiten Schiene des Tisches zu gleiten, wobei die zweite Schiene von der ersten Schiene getrennt ist oder mit ihr zusammenfällt, wobei ein zweites Ende der ersten Stange (102) an dem ersten Schlitten (104) angelenkt ist;
wobei ein zweites Ende der zweiten Stange (103) an dem zweiten Schlitten (105) angelenkt ist; und ferner umfassend Mittel (106), die dazu geeignet sind, die Drehung der ersten Stange (102) und/oder der zweiten Stange (103) festzulegen;
**dadurch gekennzeichnet, dass** es einen Federauslöser (506) umfasst, um ein Drehmoment auf die erste oder zweite Stange aufzubringen; wobei die erste oder zweite Stange dazu geeignet ist, sich längs eines Rotationshubs zwischen einer ersten Endwinkelstellung und einer zweiten Endwinkelstellung zu drehen,
wobei der Auslöser:
- kein Drehmoment aufbringt, wenn die erste oder zweite Stange (102, 103) sich in einer vorgegebenen Zwischenwinkelstellung zwischen der ersten Endwinkelstellung und der zweiten Endwinkelstellung befindet,
- ein zu der vorgegebenen Zwischenwinkelstellung gerichtetes Drehmoment aufbringt, wenn die erste oder zweite Stange (102, 103) sich in der ersten Endwinkelstellung befindet,
- ein zu der vorgegebenen Zwischenwinkelstellung gerichtetes Drehmoment aufbringt, wenn die erste oder zweite Stange (102, 103) sich in der zweiten Endwinkelstellung befindet; und
wobei der Auslöser (506) dazu geeignet ist, Energie zu speichern, wenn die erste oder zweite Stange sich von der vorgegebenen Zwischenwinkelstellung in die erste Endwinkelstellung dreht oder wenn die erste oder zweite Stange (102, 103) sich von der vorgegebenen Zwischenwinkelstellung in die zweite Endwinkelstellung dreht.

2. Röntgengerät nach Anspruch 1, wobei die erste Stange (102) und die zweite Stange (103) direkt voneinander uneingeschränkt sind.

3. Röntgengerät nach Anspruch 1 oder 2, wobei die erste und die zweite Schiene als Einzelschiene (112) hergestellt sind.

4. Röntgengerät nach Anspruch 1 oder 2 oder 3,
wobei der erste motorbetriebene Schlitten (104) einen ersten Elektromotor umfasst; wobei der zweite motorbetriebene Schlitten (105) einen zweiten Elektromotor umfasst; wobei die Mittel (106) einen dritten Elektromotor umfassen, der mit dem ersten oder dem zweiten Elektromotor verbunden ist.

5. Röntgengerät nach Anspruch 4, wobei die Mittel (106) einen kreisförmigen Bogen eines Zahnrades umfassen, das an der Basis (101) befestigt ist und wobei der dritte Motor mit einer rotierenden Welle mit einem gezahnten Stift versehen ist, der in das Zahnrad eingreift.

6. Röntgengerät nach einem der vorstehenden Ansprüche, umfassend ferner eine computergestützte Steuereinheit, die dazu geeignet ist, den ersten Elektromotor, den zweiten Elektromotor und den dritten Elektromotor zu steuern.

7. Röntgengerät nach einem der vorstehenden Ansprüche, wobei der Röntgenstrahler indirekt in dem Tisch eingebaut ist.

8. Röntgengerät nach einem der vorstehenden Ansprüche, wobei der Röntgenstrahler direkt in dem Tisch eingebaut ist.

## Revendications

1. Appareil radiologique comprenant une base (101) adaptée pour être posée sur un sol, une source de rayons X, un récepteur de rayons X, une table (110) avec un plan pour patient (111) et un système de déplacement pour ladite table ; dans lequel ledit système de déplacement comprend :
- une première tige (102) articulée à une première extrémité au niveau d'un premier point fixé de ladite base (101),
- une deuxième tige (103) articulée à une première extrémité au niveau d'un deuxième point fixé de ladite base, ledit deuxième point étant à une distance prédéterminée et fixe dudit premier point,
- un premier chariot motorisé (104) adapté pour glisser le long d'une première guide de ladite table,
- un deuxième chariot motorisé (105) adapté pour glisser le long d'une deuxième guide de ladite table, ladite deuxième guide étant distincte ou coïncidant avec ladite première guide ; dans lequel une deuxième extrémité de ladite première tige (102) est articulée audit premier chariot (104) ;
dans lequel une deuxième extrémité de ladite deuxième tige (103) est articulée audit deuxième chariot (105) ; et comprenant en outre des moyens (106) adaptés pour contraindre la rotation de ladite première tige
(102) et/ou de ladite deuxième tige (103) ;
**caractérisé en ce qu'**il comprend un actionneur à ressort (506) adapté pour appliquer un couple sur ladite première ou deuxième tige ;
dans lequel ladite première ou deuxième tige est adaptée pour pivoter le long d'une course rotatoire entre une première fin de course angulaire et une deuxième fin de course angulaire,
dans lequel ledit actionneur :
- n'applique pas de couple quand ladite première ou deuxième tige (102, 103) est dans une position angulaire intermédiaire prédéterminée entre ladite première fin de course angulaire et ladite deuxième fin de course angulaire,
- applique un couple en direction de ladite position angulaire intermédiaire prédéterminée quand ladite première ou deuxième tige (102, 103) est dans ladite première fin de course angulaire,
- applique un couple en direction de ladite position angulaire intermédiaire prédéterminée quand ladite première ou deuxième tige (102, 103) est dans ladite deuxième fin de course angulaire, et
dans lequel ledit actionneur (506) est adapté pour accumuler de l'énergie quand ladite première ou deuxième tige pivote de ladite position angulaire intermédiaire prédéterminée à ladite première fin de course angulaire et quand ladite première ou deuxième tige (102, 103) pivote de ladite position angulaire intermédiaire prédéterminée à ladite deuxième fin de course angulaire.

2. Appareil radiologique selon la revendication 1, dans lequel ladite première tige (102) et ladite deuxième tige (103) sont directement sans contraintes l'une par rapport à l'autre.

3. Appareil radiologique selon la revendication 1 ou 2, dans lequel ladite première guide et ladite deuxième guide sont constituées d'une guide unique (112).

4. Appareil radiologique selon la revendication 1 ou 2 ou 3,
dans lequel ledit premier chariot motorisé (104) comprend un premier moteur électrique ;
dans lequel ledit deuxième chariot motorisé (105) comprend un deuxième moteur électrique ;
dans lequel lesdits moyens (106) comprennent un troisième moteur électrique accouplé avec ledit premier ou deuxième moteur électrique.

5. Appareil radiologique selon la revendication 4, dans lequel lesdits moyens (106) comprennent un arc circulaire de roue dentée fixé à ladite base (101), et dans lequel ledit troisième moteur est muni d'un arbre tournant avec un pivot denté engrené dans ladite roue dentée.

6. Appareil radiologique selon l'une quelconque des revendications précédentes, comprenant en outre une unité de commande informatisée adaptée pour contrôler ledit premier moteur électrique, ledit deuxième moteur électrique, et ledit troisième moteur électrique.

7. Appareil radiologique selon l'une quelconque des revendications précédentes, dans lequel ladite source de rayons X est montée indirectement sur ladite table.

8. Appareil radiologique selon l'une quelconque des revendications précédentes, dans lequel ledit récepteur de rayons X est monté directement sur ladite table.
